(19) Europäisches Patentamt
European Patent Office
Office européen des brevets

(11) Numéro de publication : **0 375 559 B1**

(12) **FASCICULE DE BREVET EUROPEEN**

(45) Date de publication du fascicule du brevet : **21.07.93 Bulletin 93/29**

(51) Int. Cl.$^5$ : **C07J 31/00,** A61K 31/565, C07J 41/00

(21) Numéro de dépôt : **89403613.6**

(22) Date de dépôt : **22.12.89**

(54) **Nouveaux produits stéroides comportant un radical alkylthio en position 4, leur procédé de préparation, leur application comme médicaments et les compositions pharmaceutiques les renfermant.**

(30) Priorité : **22.12.88 FR 8816993**

(43) Date de publication de la demande : **27.06.90 Bulletin 90/26**

(45) Mention de la délivrance du brevet : **21.07.93 Bulletin 93/29**

(84) Etats contractants désignés : **CH DE FR GB IT LI NL**

(56) Documents cités :
**DE-B- 1 217 375**
**GB-A- 2 166 742**
**JOURNAL OF MEDICINAL CHEMISTRY, vol. 29, no. 4, avril 1986, pages 582-584, American Chemical Society, Washington, DC, US; Y.J. ABUL-HAJJ: "Synthesis and evaluation of 4-(substituted thio)-4-androstene-3,17-dione derivatives as potential aromatase inhibitors"**
**JOURNAL OF MEDICINAL CHEMISTRY, vol. 28, no. 6, juin 1985, pages 788-795, American Chemical Society, Washington, DC, US; D.A. MARSH et al.: "Aromatase inhibitors. Synthesis and biological activity of androstenedione derivatives"**

(73) Titulaire : **ROUSSEL-UCLAF**
**35, Boulevard des Invalides**
**F-75007 Paris (FR)**

(72) Inventeur : **Gourvest, Jean-François**
**2, rue Pierre Allaire**
**F-94340 Joinville Le Pont (FR)**
Inventeur : **Lesuisse, Dominique**
**2, rue Cazotte**
**F-75018 Paris (FR)**
Inventeur : **Philibert, Daniel**
**16, rue Chevalier**
**F-94210 La Varenne St Hilaire (FR)**
Inventeur : **Vevert, Jean Paul**
**55, rue Rouget de l'Isle**
**F-93500 Pantin (FR)**

(74) Mandataire : **Bourgouin, André et al**
**Département des Brevets ROUSSEL UCLAF**
**111, route de Noisy B.P. no 9**
**F-93230 Romainville (FR)**

EP 0 375 559 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux produits stéroïdes comportant un radical alkylthio substitué en position 4, leur procédé de préparation, leur application comme médicaments et les compositions pharmaceutiques les renfermant.

Les brevets allemand DE 1,217,375 et britannique GB 2,166,742 décrivent des produits stéroïdes comportant en position 4 un radical alkylthio non substitué.

L'invention a pour objet, les produits de formule générale (I) :

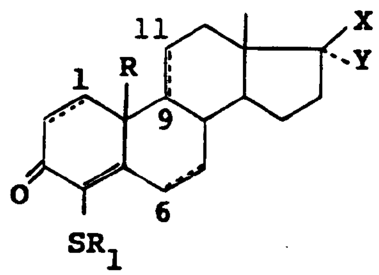

(I)

dans laquelle le substituant R représente un radical alkyle alcényle ou alcynyle ayant au plus 4 atomes de carbone ou un radical CN ;

$R_1$ représente un radical alkyle ayant de 1 à 6 atomes de carbone substitué par un ou plusieurs substituants compris dans le groupe formé par les radicaux alkyloxy ou alkylthio ayant de 1 à 4 atomes de carbone, nitro, cyano et les atomes d'halogènes ; les substituants X et Y sont tels que soit ils représentent ensemble un groupement oxo soit X représente un radical hydroxy éventuellement acylé ou éthérifié et Y représente un atome d'hydrogène, les traits pointillés en position 1(2), 6(7) et 9(11) indiquent la présence éventuelle d'une seconde liaison entre les carbones qui les portent.

Parmi les valeurs de R, on peut citer les radicaux méthyle, éthyle, propyle, butyle, isobutyle, vinyle, allyle, éthynyle, propynyle. On préfère les valeurs méthyle, éthyle, éthynyle ou propynyle. La valeur préférée est la valeur méthyle. Par propynyle on entend 1 ou 2-propynyle.

Parmi les valeurs de $R_1$, on peut citer les valeurs méthyle, éthyle, propyle, isopropyle, butyle, isobutyle, butyle secondaire, tert-butyle, pentyle, hexyle. On préfère les valeurs alkyle ayant de 1 à 4 atomes de carbone. La valeur méthyle est la valeur préférée.

Parmi les radicaux alkyloxy et alkylthio qui substituent éventuellement le radical alkyle, on peut citer les valeurs méthoxy, méthylthio, éthoxy, éthylthio, propyloxy et propylthio ainsi que n-butyl, sec-butyl ou tert-butyl oxy ou thio. On préfère les radicaux alkyloxy et alkylthio ayant 1 ou 2 atomes de carbone et de préférence encore les radicaux méthoxy et méthylthio.

Parmi les atomes d'halogènes, on peut citer des atomes de fluor, chlore, brome ou iode. On préfère les atomes de fluor ou de chlore.

Par radical hydroxy éventuellement acylé ou éthérifié on entend un radical $OR_{17}$ dans lequel $R_{17}$ représente un atome d'hydrogène, un radical acyle dérivé d'un acide organique carboxylique ayant de 1 à 18 atomes de carbone ou le reste d'un radical éther facilement éliminable. Lorsque $R_{17}$ représente un radical acyle, $R_{17}$ représente de préférence le reste d'un acide aliphatique ou cycloaliphatique saturé ou insaturé et notamment le reste d'un acide alcanoïque tel que par exemple l'acide acétique, propionique, butyrique ou isobutyrique, valérique ou undécylique, le reste d'un acide hydroxyalcanoïque tel que par exemple l'acide hydroxyacétique, le reste d'un acide cycloalkylcarboxylique ou (cycloalkyle) alcanoïque tel que par exemple l'acide cyclopropyl, cyclopentyl ou cyclohexylcarboxylique, cyclopentyle ou cyclohexyle acétique ou propionique, le reste d'un acide benzoïque ou d'un acide phénylalcanoïque tel que l'acide phényl acétique ou phényl propionique, le reste d'un amino acide tel que l'acide diéthylamino acétique ou aspartique ou le reste de l'acide formique. On préfère les radicaux acétyle, propionyle ou benzoyle. Parmi les radicaux éthers facilement éliminables on préfère les radicaux tert-butyle, benzyle ou méthoxyméthyle.

Il est entendu que le radical $R_1$ peut être monosubstitué par l'un des radicaux mentionnés ci-dessus ainsi que par les radicaux nitro et cyano mais qu'il peut être substitué par plusieurs substituants identiques ou différents. Parmi les radicaux pluri-substitués on préfère les radicaux pluri-substitués par des atomes d'halogènes tels que le radical $CHF_2$.

Parmi les valeurs de $R_1$, on préfère les radicaux du type :

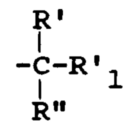

dans lesquels R' représente un atome d'hydrogène ou d'halogène, R" représente un atome d'hydrogène ou un radical alkyle ayant de 1 à 5 atomes de carbone et $R'_1$ représente un radical alkyloxy ou alkylthio ayant de 1 à 4 atomes de carbone, nitro, cyano ou un atome d'halogène.

Parmi les produits de formule (I) telle que définie ci-dessus on préfère les produits de formule (I) dans laquelle R représente un radical méthyle et $R_1$ est choisi parmi les radicaux $CH_2Hal$ ou $CHHal_2$ dans lesquels Hal représente un atome d'halogène, $CH_2CN$, $CH_2OCH_3$, $CH_2SCH_3$ et $CH_2NO_2$ et plus particulièrement encore les produits de formule (I) dans laquelle $R_1$ est choisi parmi les radicaux $CH_2Cl$, $CH_2F$ ou $CHF_2$ et R représente un radical méthyle.

Parmi les produits de formule (I), l'invention a particulièrement pour objet les produits décrits ci-après dans les exemples et en particulier :

- la 4-[(fluorométhyl) thio] androst-4-ène-3,17-dione et
- la 4-[(chlorométhyl) thio] androst-4-ène-3,17-dione.

La présente invention a aussi pour objet un procédé de préparation des produits de formule générale (I), caractérisé en ce que l'on fait agir un halogénure de formule $Hal'R_1$ dans laquelle Hal' représente un atome d'halogène sur un produit de formule (II) :

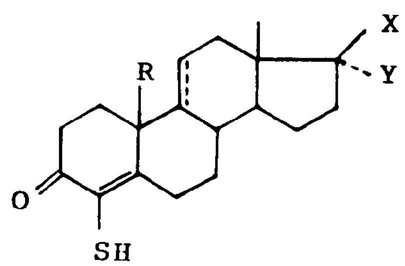

(II)

pour obtenir un produit de formule ($I_A$) :

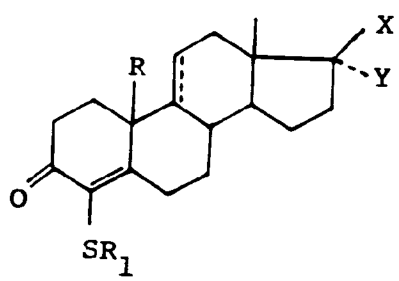

($I_A$)

correspondant à un produit de formule (I) dans laquelle les traits pointillés en position 1(2) et 6(7) représentent une simple liaison entre les carbones qui les portent et si désiré l'on fait agir, sur le produit de formule ($I_A$) un réactif de déshydrogénation pour obtenir un produit de formule ($I_B$) :

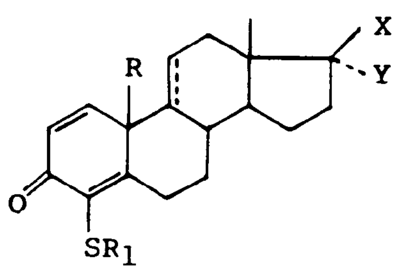

($I_B$)

correspondant à un produit de formule (I) dans laquelle le trait pointillé en position 1(2) indique la présence d'une seconde liaison entre les carbones qui le portent et le trait pointillé en position 6(7) représente une simple liaison et si désiré l'on fait agir, sur les produits de formule ($I_A$) ou ($I_B$) d'abord un réactif de protection de la fonction 3-céto delta-4 puis un réactif de déshydrogénation pour obtenir un produit de formule ($I_C$) :

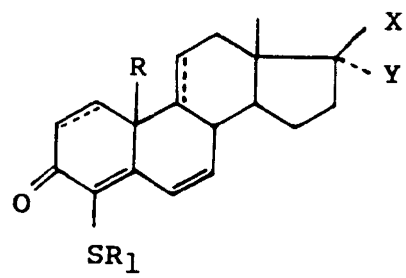

($I_C$)

correspondant à un produit de formule (I) dans laquelle le trait pointillé en position 6(7) indique la présence d'une seconde liaison entre les carbones qui le portent.

Dans des conditions préférentielles d'exécution de ce procédé, décrit ci-dessus, on opère comme suit :

- on fait d'abord agir sur le produit de formule (II) une base forte qui peut être par exemple le tert-butylate de potassium, l'hydrure de sodium ou le diisopropylamidure de lithium. On opère par exemple dans un solvant tel que le tétrahydrofuranne.
- l'halogénure Hal'$R_1$ que l'on utilise est de préférence le bromure, ou l'iodure. On peut aussi utiliser la chlorure.
- la transformation éventuelle des produits de formule ($I_A$) en produits de formule ($I_B$) est réalisée de préférence par la 2,3-dichloro 5,6-dicyano 1,4-benzoquinone (D.D.Q.).
- la transformation éventuelle des produits de formules ($I_A$) ou ($I_B$) en produits de formule ($I_C$) est réalisée de préférence par action d'abord d'un orthoformiate tel que l'orthoformiate d'éthyle en présence par exemple, d'acide para-toluènesulfonique dans l'éthanol ou un solvant usuel, puis par action du chloranile dans un solvant tel que l'acétone aqueux.

Les produits de formule ($I_A$) dans laquelle le substituant $R_1$ représente un radical alkyle non substitué peuvent également être préparés en faisant agir sur un produit de formule (IV) :

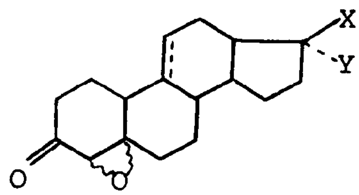

(IV)

un dérivé d'un thiol de formule

$$R'_1SH$$

dans laquelle $R'_1$ représente un radical alkyle non substitué, au sein d'un alcool inférieur.

Dans des conditions préférentielles d'exécution de ce procédé, le dérivé du thiol est un thiolate alcalin tel que le méthanethiolate de sodium et la réaction est effectuée au sein du méthanol.

L'observation selon laquelle environ 35 % des cancers du sein sont estrogéno dépendants a conduit à rechercher les moyens de limiter la production d'estrogènes.

Après avoir utilisé des méthodes chirurgicales consistant à supprimer les sources d'estrogènes (ovaires) ou les sources de leurs précurseurs biosynthétiques, les androgènes (glandes surrénales) on a cherché à développer des méthodes moins traumatisantes.
(ABUL-HAJJ Y.J.O. Steroid Biochem 13 (1980), 1935 ; BRODIE A.M.H. Cancer Res. 42, (1982), 3312).

A cet égard l'inhibition spécifique du dernier stade enzymatique de l'aromatisation des androgènes 3-céto delta-4 en estrogènes phénoliques, semble le moyen le plus efficace et le moins perturbant. L'enzyme responsable de cette transformation est une mono-oxygénase connue comme étant un cytochrome P450 : l'AROMATASE (BRODIE A.M.H. J. Endocrinol. Invest. 2 (1979) 445) qui requiert de l'oxygène et la NADPH (Nicotinamide adénine Dinucleotide phosphate réduit) pour effectuer l'aromatisation des androgènes en estrogènes.

Sur la base d'un autre mécanisme, d'autres auteurs (par exemple MARCOTTE et Al, Biochemistry 21,

(1982) 2773, FLYNN et Al Biochem. Biophys. Res. Com. 103 (1981) 713) ont proposé des inhibiteurs suicides pour l'Aromatase. Des inhibiteurs compétitifs tels que l'Aminogluthétimide ont également été proposés dans le traitement des cancers métastasiques du sein. Ce produit s'est cependant révélé comme n'étant pas spécifique de l'Aromatase : il s'attaque en effet à d'autres processus enzymatiques que celui conduisant des androgènes aux estrogènes.

Les produits objets de la présente invention présentent au contraire une activité spécifique du type inhibiteurs suicides de l'aromatase (cytochrome P450 aromatase).

Cette propriété inhibitrice de l'aromatase rend les produits de la présente invention aptes à être utilisés dans les affections suivantes :

- cancers du sein, de l'endomètre, de l'ovaire et du pancréas,
- gynécomasties,
- troubles bénins du sein,
- endométriose,
- affections polycystiques de l'ovaire,
- hyperplasie prostatique et plus généralement dans le traitement des hyperestrogènémies.

L'invention a donc pour objet, à titre de médicaments, les produits de formule générale (I) telle que décrite ci-dessus.

L'invention a plus spécialement pour objet, à titre de médicaments, les produits de formule générale (I), dans laquelle R représente un radical méthyle et $R_1$ est choisi parmi les radicaux $CH_2Hal$ ou $CHHal_2$ dans lesquels Hal représente un atome d'halogène, $CH_2CN$, $CH_2OCH_3$, $CH_2SCH_3$ et $CH_2NO_2$, ainsi que les produits de formule générale (I) dans laquelle $R_1$ est choisi parmi les radicaux $CH_2Cl$, $CH_2F$ ou $CHF_2$ et R représente un radical méthyle.

L'invention a tout spécialement pour objet, à titre de médicament :

- la 4-[(fluorométhyl) thio] androst-4-ène-3,17-dione et
- la 4-[(chlorométhyl) thio] androst-4-ène-3,17-dione.

La posologie varie en fonction de l'affection à traiter et de la voie d'administration. Elle peut varier par exemple de 0,1 à 50 mg/kg de préférence 0,5 à 10 mg/kg et par jour chez l'adulte par voie orale.

Les nouveaux produits de formule (I) peuvent être employés pour préparer des compositions pharmaceutiques renfermant, à titre de principe actif, l'un au moins desdits produits.

Les produits de formule (I) sont utilisés par voie digestive, parentérale ou locale par exemple par voie transdermique. Ils peuvent être prescrits sous forme de comprimés simples ou dragéifiés, de gélules, de granulés, de suppositoires, d'ovules, de préparations injectables, de pommades, de crèmes, de gels, de patches, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a donc pour objet les compositions pharmaceutiques renfermant comme principe actif au moins un produit de formule (I).

Certains des produits de formule (II) et (IV) utilisés au départ du procédé de l'invention sont connus. C'est ainsi que le 4-thioandrostènedione et le dérivé 4,5-époxy correspondant utilisé au départ des produits décrits ci-après dans les exemples sont décrits dans les publications suivantes :

- J. Med. Chem. 28, 788 (1985)
- J. Med. Chem. 29, 582 (1986).

Les produits de formule (II) et (IV) qui ne sont pas connus peuvent être préparés comme suit :

On fait agir sur un produit de formule (III) :

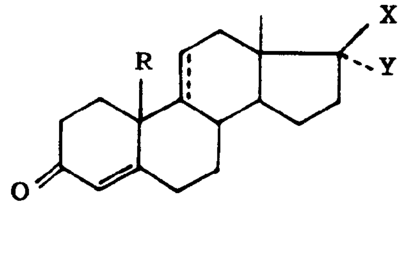

(III)

un agent d'époxydation qui peut être par exemple, l'eau oxygénée en présence de soude dans un mélange de solvant tel que le chlorure de méthylène et le méthanol ou le péroxyde de tert-butyle en présence de triton B

dans le méthanol, pour obtenir un produit de formule (IV) :

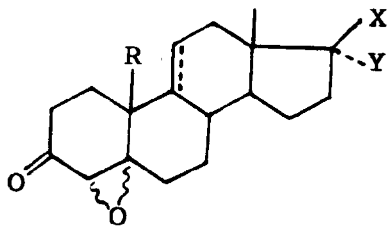

(IV)

sur lequel on fait agir l'acide thioacétique dans un solvant tel que le dioxanne ou le méthanol puis,
soit un acide tel que l'acide chlorhydrique dans un solvant tel que le méthanol, le tétrahydrofuranne, l'acétone,
soit un alcoolate alcalin tel que le méthylate de sodium dans le méthanol,
soit la potasse dans le méthanol, pour obtenir le produit de formule (II).

Les produits de formule (III) à leur tour sont décrits ou sont accessibles par des méthodes connues de l'homme du métier.

Des produits de formule (III) possédant une double liaison en position 9(11) sont décrits, notamment, dans les références suivantes :
R = alkyle : Brevet français 1.255.101 ; Brevet britannique GB 1.081.307 ; Bull. Soc. Chim-Fr. 1970 (7) p. 2556-64.
R = alcényle : Brevet américain USP 3.284.448. Steroïds 1981, 37 (4), 361-82.
R = alcynyle : Brevet allemand 36.44.358, J. Biol. Chem. 1981, 256 (3), 1076-9.
Les produits de formule (III) ne possèdant pas de double liaison en position 9(11) sont décrits, notamment dans les références suivantes :
R = alkyle, alcényle, alcynyle et CN : Steroïds 1982, 39 (3) p. 325-44.
R = alcynyle : Brevet américain USP 3.218.316.

En plus des produits décrits dans les exemples, qui illustrent l'invention sans toutefois la limiter, les produits suivants constituent des produits pouvant être obtenus dans le cadre de la présente invention, les substituants R, $R_1$, X et Y sont ceux indiqués dans la formule (I).

| R | $R_1$ | X | Y | 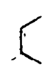 9 | |
|---|---|---|---|---|---|
| C≡C-H | $CH_2Cl$ | C=O | | 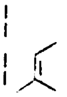 | |
| $CH_3$ | " | " | | " | |
| $CH_2$-C≡CH | " | " | | " | |

**EXEMPLE 1 : 4-[(fluorométhyl) thio] androst-4-ène-3,17-dione.**

A un mélange de 2 g de 4-thioandrostène 3,17-dione en solution dans 80 $cm^3$ de tétrahydrofuranne, à 0°C, on additionne 1 g de tert-butylate de potassium. On agite durant 30 minutes à 0°C. On fait passer du bromofluorométhane dans la solution de thiolate pendant 2 heures à 20°C à l'aide d'azote comme gaz vecteur. On verse sur 80 $cm^3$ d'une solution saturée de chlorure d'ammonium et 120 $cm^3$ de dichlorométhane. On extrait au dichlorométhane, sèche et concentre. Le produit brut est chromatographié sur silice (éluant : acétate d'éthyle-Hexane (3:7)). On obtient de cette façon le produit attendu (RF = 0,56, dans AcOEt-Hexane 1:1). On le met

en solution dans du dichlorométhane et traite à chaud avec du charbon actif. On obtient 457 mg (21 %) d'une huile jaune qui cristallise. Une recristallisation dans l'éther diéthylique fournit 210,6 mg de produit attendu. F = 129°C.

Analyses

- RMN ($CDCl_3$, 300 MHz) 0,93 (18-Me), 1,28 (19-Me), 3,77 (dt, H6éq), 5,49 (d, $SCH_2F$, J=53 Hz).
- IR ($CHCl_3$) 1735 $cm^{-1}$ (17-cétone), 1675 et 1560 $cm^{-1}$(3-cétone).
- Microanalyse : $C_{20}H_{27}O_2SF$

| | | | | |
|---|---|---|---|---|
| Calculé : | C% 68,54 | H% 7,76 | S% 9,15 | F% 5,42 |
| Trouvé : | 68,8 | 8,0 | 9,0 | 5,2 |

Préparation du bromofluoroacétate utilisé au départ de l'exemple 1.

On mélange 25 g d'acide fluoroacétique, 250 $cm^3$ d'acétone et 50 $cm^3$ d'eau. On additionne en 5 minutes 25 $cm^3$ d'ammoniaque 22°Bé. On agite durant 20 minutes à température ambiante. Le mélange est ensuite amené à sec sous pression réduite. On reprend par 50 $cm^3$ d'acétone et 20 $cm^3$ d'eau et évapore à nouveau. Le solide est remis en suspension dans 500 $cm^3$ d'acétone et on additionne 100 $cm^3$ d'eau. On refroidit à 5°C puis ajoute sous agitation 54,4 g de nitrate d'argent. Le mélange est agité durant 1 heure à 0-5°C, filtré, lavé à l'acétone, puis à l'éther et séché sous pression réduite à 70°C à l'abri de la lumière. On obtient 57 g de fluoroacétate d'argent utilisé tel quel pour la réaction suivante. On mélange 6,3 g de sel d'argent, préparé ci-dessus, 100 $cm^3$ de tétrachlorure de carbone et 1,8 $cm^3$ de brome (0,031 mm). On agite à reflux (environ 64°C) pendant 2 heures 30 minutes jusqu'à fin de dégagement de $CO_2$. On refroidit à 0°C.

**EXEMPLE 2 : 4-(chlorométhylthio) androst-4-ène-3,17-dione**

On mélange 1 g de 4-thioandrostène 3,17-dione, 50 $cm^3$ de tétrahydrofuranne à 0°C et on ajoute 422,6 mg de tert-butylate de potassium. La solution est agitée 15 minutes à 0°C. On additionne ensuite 0,43 mg de bromochlorométhane, le mélange est agité 15 minutes à température ambiante. On additionne 10 $cm^3$ d'une solution saturée de chlorure d'ammonium et extrait le mélange au dichlorométhane. Les extraits organiques sont séchés, concentrés et chromatographiés (mélange éluant : AcOEt-Hexane 3:7 puis 1:1). Une première fraction (Rf=0,63 dans AcOEt-Hexane 1:1) (100 mg, 9,6 %) est du androsta-3,5-diéno[4,3-d][1,3-oxathiole]-17-one. La fraction suivante (Rf=0,48) fournit après une recristallisation dans l'éther éthylique fournit 420 mg (36 %) de produit attendu.

Analyses

- RMN ($CDCl_3$, 90Mhz) 0,96 (s, 18-Me), 1,3 (s, 19-Me), 3,65-3,81 (m, H6) 4,81 (s, $CH_2S$).
- IR ($CHCl_3$) 1734 (17-cétone), 1675, 1555 (cétone conjuguée).
- SM 366 (M+), 330 (M-HCl), 315, 300.
  F = 174°C.
- Microanalyse : $C_{20}H_{27}O_2SCl$

| | | | |
|---|---|---|---|
| Calculé : | H% 65,5 | H% 7,42 | S% 8,74 |
| Trouvé : | 65,8 | 7,7 | 8,2 |

Analyses de l'androsta-3,5-diéno[4,3-d][1,3-oxathiole]-17-one

- RMN ($CDCl_3$, 90Mhz) 0,92 (s, 18-Me), 1,03 (s, 19-Me) 5,16(H5), 5,51 ($CH_2S$).
- IR ($CHCl_3$) 1734 (17-cétone), 1657, 1634 (système conjugué).

**EXEMPLE 3 : 4-[(méthylthio) méthylthio] androst-4-ène-3,17-dione.**

On mélange 1 g de 4-thioandrostène 3,17-dione, 40 $cm^3$ de tétrahydrofuranne, refroidit le mélange à 0°C et additionne à cette température 387 mg de tert-butylate de potassium. On agite 30 minutes à 0°C. On addi-

tionne ensuite 0,313 $cm^3$ de chlorométhyle thiométhyle éther. On agite le mélange 30 minutes à température ambiante, verse dans 40 $cm^3$ d'une solution saturée de chlorure d'ammonium et 60 $cm^3$ de dichlorométhane. On extrait au dichlorométhane, sèche et concentre. Le produit brut est chromatographié sur silice (éluant : AcOEt-Hexane (3:7)). On obtient 560 mg de produit attendu sous forme d'une huile jaune. Une cristallisation dans l'éther diéthylique fournit 260 mg de produit attendu.

Analyses

- RMN ($CDCl_3$, 300 MHz) 0,93 (18-Me), 1,28 (19-Me) 2,14 (s, SMe), 3,82 (AB, $SCH_2S$).
- IR ($CHCl_3$) 1735 et 1406 $cm^{-1}$ (17-cétone), 1673 et 1557 (3-cétone).
- Microanalyse : $C_{11}H_{30}O_2S_2$

| | | | |
|---|---|---|---|
| Calculé : | C% 66,62 | H% 7,99 | S% 16,94 |
| Trouvé : | 66,7 | 8,0 | 16.6 |

**EXEMPLE 4 : 4-[(méthoxyméthyl) thio] androst-4-ène-3,17-dione**

On mélange 1 g de 4-thioandrostène 3,17-dione, 40 $cm^3$ de tétrahydrofuranne, refroidit le mélange à 0°C et ajoute à cette température 387 mg de tert-butylate de potassium. On agite 30 minutes à 0°C. On additionne ensuite 0,308 $cm^3$ de bromométhyle méthyle éther. On agite le mélange 30 minutes à température ambiante, verse dans 40 $cm^3$ d'une solution saturée de chlorure d'ammonium et 60 $cm^3$ de dichlorométhane. On extrait la phase aqueuse au dichlorométhane, sèche et concentre. Le produit brut est chromatographié sur silica (éluant : AcOEt-Hexane (3:7)). Le produit obtenu (RF = 0,5, AcOEt-Hexane 1:1) sous forme d'une huile jaune est cristallisé dans l'éther diéthylique. On obtient 109 mg de produit attendu.

Analyses

- RMN ($CDCl_3$, 300 MHz) 0,93 (18-Me) 1,26 (19-Me) 3,74 (s, OMe) 3,76 (ddd, H6équat.) 4,69 et 4,73 (2d, $SCH_2O$).
- IR ($CHCl_3$) 1735 $cm^{-1}$ (17-cétone) 1674 et 1558 (3-cétone).
- Microanalyse : $C_{11}H_{30}O_3S$

| | | | |
|---|---|---|---|
| Calculé : | C% 69,57 | H% 8,34 | S% 8,94 |
| Trouvé : | 69,5 | 8,1 | 8,6 |

**EXEMPLE 5 : 4-(difluorométhylthio) androst-4-ène-3,17-dione**

On mélange 1 g de 4-thioandrostène 3,17-dione et 20 $cm^3$ de tétrahydrofuranne, refroidit le mélange à 0°C et additionne à cette température, 422,6 mg de tert-butylate de potassium. On agite durant 5 minutes à 0°C. On introduit ensuite une quantité importante de fréon en une fois. L'excédent de fréon remplit le ballon de baudruche vide relié à l'appareil. On agite le mélange durant 12 heures à température ambiante. Après concentration, on chromatographie sur silice (éluant : AcOEt-Hexane 3:7, suivi de 1:1). On obtient 610 mg de produit attendu. Une recristallisation dans l'acétate d'éthyle fournit 400 mg de composé attendu, sous forme de cristaux.

Analyses

- RMN ($CDCl_3$, 250 Mhz) 0,93 (s, 18-Me) 1,29 (s, 19-Me) 6,91 (t, J=59Hz, $CHF_2$) 3,68 (H6).
- IR ($CHCl_3$) 1735 (17-cétone) 1680, 1559 (cétone conjuguée) 1050-1070 (C-F).
- SM 368 (M+), 348 (M-F) 317 (M-$CHF_2$) 285 (M-$SCHF_2$) 267
- PF = 129°C.
- Microanalyse : $C_{20}H_{26}O_2SF_2$

| | | | | |
|---|---|---|---|---|
| Calculé : | C% 65,19 | H% 7,11 | S% 8,7 | F% 10,31 |
| Trouvé : | 65,4 | 7,2 | 8,4 | 9,6 - 9,9 |

**EXEMPLE 6 : [(3,17-dioxo 4-androstène-4-yl) thio] acétonitrile**

On mélange 1 g de 4-thioandrostène 3,17-dione, 40 $cm^3$ de tétrahydrofuranne, refroidit à 0°C et ajoute à cette température 387 mg de tert-butylate de potassium. On agite 30 minutes à 0°C. On ajoute ensuite 0,263 mg de bromoacétonitrile et agite 30 minutes à température ambiante. On verse dans 40 $cm^3$ d'une solution saturée de chlorure d'ammonium et 60 ml de dichlorométhane. La phase aqueuse est extraite avec du dichlorométhane, séchée et concentrée. Le produit brut est chromatographié sur silice (éluant : AcOEt-Hexane (3:7)). On obtient de cette façon 640 mg de produit attendu sous forme d'une huile. Une cristallisation dans l'éther diéthylique fournit 260 mg de produit attendu.

Analyses

- RMN ($CDCl_3$, 300 MHz) 0,93 (18-Me) 1,31 (19-Me) 3,51 et 3,60 (2d, J=17Hz, $SCH_2CN$) 3,75 (dt, H-6équat.).
- IR ($CHCl_3$) 2232 $cm^{-1}$ (CN), 1735 $cm^{-1}$ (17-cétone) 1675 et 1556 (3-cétone).
- Microanalyse : $C_{11}H_{27}O_2NS$

| | | | | |
|---|---|---|---|---|
| Calculé : | C% 70,55 | H% 7,61 | S% 8,97 | N% 3,92 |
| Trouvé : | 70,7 | 7,7 | 8,7 | 3,9 |

**EXEMPLE 7 : 4-[(nitrométhyl) thio] androst-4-ène-3,17-dione**

On mélange 1 g de 4-thioandrostène 3,17-dione, 40 $cm^3$ de tétrahydrofuranne, refroidit à 0°C et ajoute à cette température 387 mg de tert-butylate de potassium. On agite 30 minutes à 0°C. On ajoute ensuite 0,263 $cm^3$ de bromonitrométhane, agite 30 minutes à température ambiante et verse dans 40 $cm^3$ d'une solution saturée de chlorure d'ammonium et 60 $cm^3$ de dichlorométhane. La phase aqueuse est extraite avec du dichlorométhane séchée et concentrée. Le produit brut est chromatographié sur silice (éluant : d'AcOEt-Hexane (3:7)). On obtient de cette façon 134 mg de produit attendu.

Analyses

- RMN ($CDCl_3$, 200 MHz) 0,93 (18-Me) 1,28 (19-Me) 3,61 (ddd, H6équat.) 5,34 (AB, J=12,5Hz, $SCH_2NO_2$).
- IR ($CHCl_3$) 1735 $cm^{-1}$ (17-cétone) 1677 et 1553 (3-cétone).

**EXEMPLE 8 : 4-(méthylthio) androsta 4,9(11-dièn-3,17-dione.**

On ajoute 39,2 $cm^3$ d'eau oxygénée à 30% dans 23 g d'androsta 4,9(11)-dièn-3,17-dione en solution dans 200 $cm^3$ de chlorure de méthylène additionné de 2 litres de méthanol. On agite 30 minutes à température ambiante, refroidit à 0°C, ajoute en 30 minutes 8,5 $cm^3$ de soude 4 M et maintient sous agitation 12 heures à 4°C. On neutralise la réaction par addition d'acide chlorhydrique N, évapore partiellement le solvant jusqu'à 200 $cm^3$ environ, ajoute 200 $cm^3$ d'eau, et filtre le précipité obtenu. Après lavage à l'éther, on obtient 16,8 g de produit 4,5-époxyde. Rf = 0,44 (cyclohexane-acétate d'éthyle 1-1). On ajoute 232 mg de méthanethiolate de sodium à 1 g de l'époxyde obtenu ci-dessus dans 400 $cm^3$ de tétrahydrofuranne et agite 1 heure à température ambiante. On verse le milieu réactionnel dans l'eau, filtre le précipité et recueille 420 mg de produit brut. On extrait le filtrat au chlorure de méthylène, sèche, concentre à sec et recueille 600 mg de produit brut. On réunit les 2 unités de produit brut et les purifie par chromatographie sous pression (éluant : cyclohexane-acétate d'éthyle 8-2) et récupère 500 mg de produit attendu. Rf = 0,51 (acétate d'éthyle-cyclohexane 1-1).

Spectre IR ($CHCl_3$)

C=O (17 céto) : 1735 $cm^{-1}$

cétone conjugué 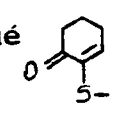 : 1673 $cm^{-1}$

1556 $cm^{-1}$

**EXEMPLE 9 : 4-méthylthio androsta 4,5,9(11)-trièn-3,17-dione.**

On agite pendant 2 heures à température ambiante 300 mg de produit obtenu à l'exemple 8 dans 7,5 $cm^3$ d'éthanol, 1,1 $cm^3$ d'orthoformiate d'éthyle en présence de 1,5 mg d'acide para-toluènesulfonique. On ajoute alors 0,4 $cm^3$ de triéthylamine, verse le milieu réactionnel dans 50 $cm^3$ d'une solution aqueuse saturée de bicarbonate de sodium, extrait au chlorure de méthylène, sèche, ajoute 0,5 $cm^3$ de triéthylamine et élimine le solvant. On obtient 200 mg de produit 3-éthoxy. On ajoute 250 mg de chloranile aux 200 mg de produit obtenu ci-dessus dans 9,3 $cm^3$ d'un mélange acétone-eau (95-5). On agite 16 heures à température ambiante, verse dans 10 $cm^3$ d'une solution de thiosulfate de sodium à 10%, ajoute 10 $cm^3$ d'une solution aqueuse de bicarbonate de sodium et agite 1 heure à température ambiante. On extrait au chlorure de méthylène, sèche, évapore le solvant, purifie le résidu par chromatographie sous pression (éluant : acétate d'éthyle-cyclohexane 2-8) et obtient 100 mg de produit attendu. Rf = 0,61 (acétate d'éthyle-cyclohexane 1-1).

Spectre IR ($CHCl_3$)

| | |
|---|---|
| (17 céto) : | 1735 $cm^{-1}$ |
| | 1667 $cm^{-1}$ |
| cétone conjugué | 1616 $cm^{-1}$ |
| | 1525 $cm^{-1}$ |

**EXEMPLE 10 : 4-méthylthio androsta 1,4,9(11)-trièn-3,17-dione.**

On ajoute 172 mg de 2,3-dichloro 5,6-dicyano 1,4-benzoquinone (D.D.Q.) à 100 mg de produit obtenu à l'exemple 8 dans 30 $cm^3$ de dioxane et chauffe 16 heures au reflux. On évapore le solvant, élimine le D.D.Q. par filtrat sur silice, purifie par chromatographie sous pression (éluant : acétate d'éthyle-cyclohexane 2-8) et obtient 30 mg de produit attendu. Rf = 0,49 ( acétate d'éthyle-cyclohexane 1-1).

**EXEMPLE 11 : 4-méthylthio androsta 1,4,6,9(11)-tétraèn-3,17-dione.**

On ajoute 5,5 g de D.D.Q. à 3,2 g de produit obtenu comme à l'exemple 8 dans 350 $cm^3$ de dioxane et agite 24 heures à température ambiante puis 12 heures au reflux. On évapore le dioxane, filtre sur silice, reprend le produit dans 350 $cm^3$ de dioxane, ajoute 5,5 g de D.D.Q. et chauffe de nouveau 12 heures au reflux. On évapore le dioxane, filtre sur silice pour éliminer le D.D.Q., purifie par chromatographie sur silice (éluant : cyclohexane-acétate d'éthyle 8-2) et obtient 2,5 g de produit brut que l'on recristallise dans l'éther. Le filtrat obtenu au cours de la synthèse permet d'obtenir 2,31 g d'un mélange (1-1) de produit delta 1,4,6,9(11)-tetraène et de delta 1,4,9(11)-triène.

Analyse : $C_{20}H_{22}O_2S$

| | | | | |
|---|---|---|---|---|
| Calculé | : | C% 73,5 | H% 6,85 | S% 9,82 |
| Trouvé | : | 73,4 | 6,8 | 9,8 |

**EXEMPLE 12 : 4-[(fluorométhyl)thio] androsta 4,6,9(11)-trièn-3,17-dione.**

On ajoute à 0°C 160 mg d'acide métachloroperbenzoïque à 300 mg de produit obtenu à l'exemple 9 dans 10 $cm^3$ de chlorure de méthylène et agite 30 minutes à 0°C. On ajoute de nouveau 16 mg d'acide, agite 10 minutes à 0°C puis ajoute 5 $cm^3$ d'une solution aqueuse saturée en bicarbonate de sodium. On extrait au chlorure de méthylène, sèche et concentre à sec. On recueille 310 mg de sulfoxyde. Rf = 0,06 (acétate d'éthyle-cyclohexane 1-1). On refroidit à -78°C 170 mg de sulfoxyde dans 5 $cm^3$ de chloroforme, ajoute 0,14 $cm^3$ de trifluorure de diéthylaminosulfure, agite 7 heures à température ambiante puis 16 heures au reflux. On refroidit, ajoute 5 $cm^3$ de solution aqueuse de bicarbonate de sodium, extrait au chlorure de méthylène, sèche et évapore le solvant. On purifie le résidu par chromatographie sur silice sous pression (éluant : acétate d'éthyle-cyclohexane 3-7) et obtient 75 mg de produit attendu. Rf = 0,6 (acétate d'éthyle-cyclohexane 1-1).

**Compositions pharmaceutiques** :

On a préparé des comprimés répondant à la formule suivante :

- Produit de l'exemple 1 50mg
- Excipient q.s. pour un comprimé terminé à 120 mg

(Détail de l'excipient : talc, amidon, stéarate de magnésium).

**Etude pharmacologique des produits de l'invention.**

Inhibition dépendante de la concentration (mesure de la $CI_{50}$ = concentration de l'inhibiteur nécessaire pour réduire l'activité enzymatique de 50 %).

On utilise des placentas humains qui une heure au plus après l'accouchement sont lavés, perfusés par du sérum physiologique (5 litres) via la veine ombilicale puis congelés à : -40°C.

1) Obtention des microsomes placentaires

Les placentas sont décongelés à 4°C puis homogénéisés (1:3) dans un tampon phosphate 10 mM, pH = 7,0 ; contenant 100 millimoles de chlorure de potassium (KC1), 10 millimoles de dithiothreitol (DTT), 10 millimoles d'acide éthylènediaminetétraacétique (EDTA), 40 millimoles de nicotinamide et 250 millimoles de sucrose.
Les homogénats sont ensuite soumis à différentes phases de centrifugation jusqu'à l'obtention du surnageant "9000 g" (correspondant au cytosol et au réticulum endoplasmique).
Ce surnageant est alors soumis à une étape d'ultracentrifugation (1 heure 30 minutes, 105000 g) pour obtenir le culot microsomal.
Les microsomes sont alors resuspendus dans un tampon phosphate 50 millimoles, pH = 7,4, contenant 100 millimoles KC1, 1 millimole EDTA, 1 millimole DTT et du glycérol (10 %).
La suspension microsomale est ensuite aliquotée et les fractions congelées à la température de l'azote liquide.
La concentration en protéines de la suspension microsomale est déterminée par la méthode de BRADFORD (BRADFORD M., Anal. Biochem., 72, (1976) 248).

2) Mesure de la $CI_{50}$ de chaque inhibiteur

A 960 microlitres de tampon phosphate (50 millimoles, pH = 7,2), 2,5 millimoles glucose-6-phosphate, et contenant 0,16 unité internationale de glucose-6-phosphate déshydrogénase (G-6-PDH), on ajoute dans l'ordre :

1° - 10 microlitres d'inhibiteur, solubilisé dans le diméthylsulfoxyde (DMSO), pour donner des concentrations finales allant de $10^{-6}$M à $10^{-9}$M.

2° - 10 microlitres de substrat. Le substrat est de l'Androstènedione 60 nM solubilisée dans l'éthanol et contenant de la 1béta-2béta-($^3$H)-Androstènedione à dilution isotopique connue (-~ 200.000 désintégrations par minute).

3° - 10 microlitres de suspension microsomale, équivalant à 25 microgrammes de protéines par test.

La réaction enzymatique est alors très rapidement initiée par un ajout de 10 microlitres de nicotinamide adénine dinucléotide phosphate réduit (NADPH) solubilisé dans l'eau.
Après agitation, chaque test est incubé à 37°C pendant 10 minutes. La réaction est ensuite stoppée par un ajout de chloroforme (4 $cm^3$).
Après agitation vigoureuse des tubes, ceux-ci sont décantés et centrifugés à 4°C pendant 10 minutes à la vitesse de 3000 r.p.m. (rotations par minutes) soit 600 X g.
Après centrifugation, et pour chaque tube, 100 microlitres de surnageant sont prélevés et mis à compter en présence de liquide scintillant.
Cette méthode est dérivée des procédures décrites par REED et Coll. (J.Biol. Chem., 251, (1976), 1625) et THOMPSON et Coll. (J.Biol. Chem., 249, (1974), 5364).
L'activité enzymatique (aromatase), est proportionnelle au pourcentage de tritium relargué sous forme d'eau tritiée ($^3H_2O$) au cours de la réaction.
L'inhibition obtenue pour chaque concentration de chaque produit inhibiteur de l'invention est calculée comme un pourcentage des contrôles (100 % arbitraire, obtenus en absence de tout inhibiteur).
La $CI_{50}$ est égale à la concentration d'inhibiteur nécessaire pour diminuer de 50 % l'activité enzymatique.
Les valeurs des $CI_{50}$ obtenues pour les produits inhibiteurs de l'invention sont les suivantes :
Produit de l'exemple 1 : $CI_{50}$ = 0,07 micromole
Produit de l'exemple 2 : $CI_{50}$ = 0,075 micromole.

## Revendications

1. Les produits de formule générale (I) :

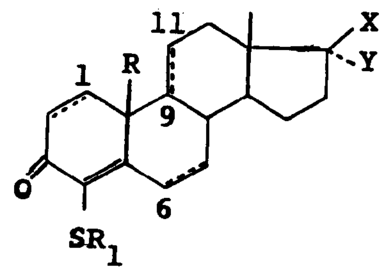

(I)

dans laquelle le substituant R représente un radical alkyle alcényle ou alcynyle ayant au plus 4 atomes de carbone ou un radical CN ;
$R_1$ représente un radical alkyle ayant de 1 à 6 atomes de carbone substitué par un ou plusieurs substituants compris dans le groupe formé par les radicaux alkyloxy ou alkylthio ayant de 1 à 4 atomes de carbone, nitro, cyano et les atomes d'halogènes ; les substituants X et Y sont tels que soit ils représentent ensemble un groupement oxo soit X représente un radical hydroxy éventuellement acylé ou éthérifié et Y représente un atome d'hydrogène, les traits pointillés en position 1(2), 6(7) et 9(11) indiquent la présence éventuelle d'une seconde liaison entre les carbones qui les portent.

2. Les produits de formule générale (I) telle que définie à la revendication 1 dans laquelle R représente un radical méthyle et $R_1$ est choisis parmi les radicaux $CH_2Hal$ ou $CHHal_2$ dans lesquels Hal représente un atome d'halogène, $CH_2CN$, $CH_2OCH_3$, $CH_2SCH_3$ et $CH_2NO_2$.

3. Les produits de formule générale (I) telle que définie à l'une des revendications 1 ou 2 dans laquelle $R_1$ est choisi parmi les radicaux $CH_2Cl$, $CH_2F$ ou $CHF_2$ et R représente un radical méthyle.

4. Les produits de formule générale (I) telle que définie à l'une des revendications 1 à 3 et répondant aux formules suivantes :
   - la 4-[(fluorométhyl) thio] androst-4-ène-3,17-dione et
   - la 4-[(chlorométhyl) thio] androst-4-ène-3,17-dione.

5. Procédé de préparation des produits de formule générale (I) telle que définie à la revendication 1 caractérisé en ce que l'on fait agir un halogénure de formule Hal'$R_1$ dans laquelle Hal' représente un atome d'halogène sur un produit de formule (II) :

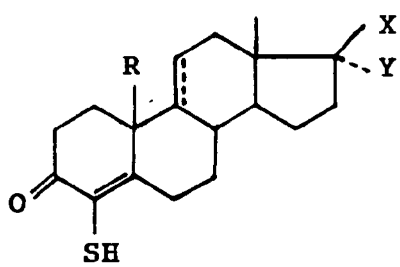

(II)

pour obtenir un produit de formule ($I_A$) :

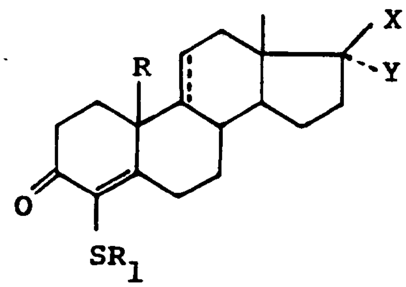

$(I_A)$

correspondant à un produit de formule (I) dans laquelle les traits pointillés en position 1(2) et 6(7) représentent une simple liaison entre les carbones qui les portent et si désiré l'on fait agir, sur le produit de formule $(I_A)$ un réactif de déshydrogénation pour obtenir un produit de formule $(I_B)$ :

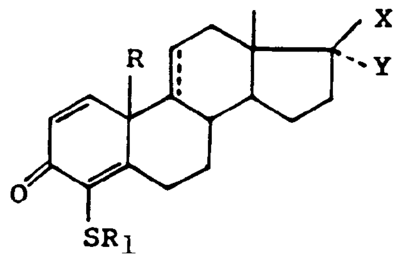

$(I_B)$

correspondant à un produit de formule (I) dans laquelle le trait pointillé en position 1(2) indique la présence d'une seconde liaison entre les carbones qui le portent et le trait pointillé en position 6(7) représente un simple liaison et si désiré l'on fait agir, sur les produits de formule $(I_A)$ ou $(I_B)$ d'abord un réactif de protection de la fonction 3-céto delta-4 puis un réactif de déshydrogénation pour obtenir un produit de formule $(I_C)$ :

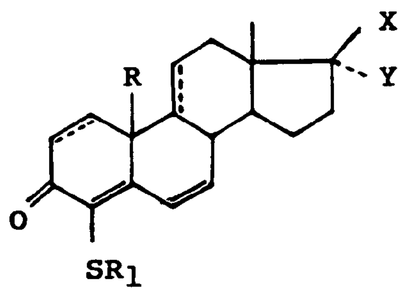

$(I_C)$

correspondant à un produit de formule (I) dans laquelle le trait pointillé en position 6(7) indique la présence d'une seconde liaison entre les carbones qui le portent.

**6.** A titre de médicaments, les produits de formule générale (I) telle que décrite à la revendication 1.

**7.** A titre de médicaments, les produits de formule générale (I) telle que décrite à l'une des revendications 2, 3 ou 4.

**8.** Les compositions pharmaceutiques renfermant, à titre de principe actif l'un au moins des médicaments définis à l'une des revendications 6 ou 7.

**9.** Utilisation des produits de formule générale (I) :

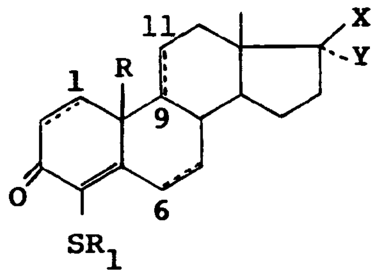

(I)

dans laquelle le substituant R représente un radical alkyle alcényle ou alcynyle ayant au plus 4 atomes de carbone ou un radical CN ;
$R_1$ représente un radical alkyle ayant de 1 à 6 atomes de carbone non substitué ou substitué par un ou plusieurs substituants compris dans le groupe formé par les radicaux alkyloxy ou alkylthio ayant de 1 à 4 atomes de carbone, nitro, cyano et les atomes d'halogènes ; les substituants X et Y sont tels que soit ils représentent ensemble un groupement oxo soit X représente un radical hydroxy éventuellement acylé ou éthérifié et Y représente un atome d'hydrogène, les traits pointillés en position 1(2), 6(7) et 9(11) indiquent la présence éventuelle d'une seconde liaison entre les carbones qui les portent, étant entendu que lorsque $R_1$ représente un radical alkyle non substitué et X et Y représentent ensemble un groupement oxo, l'un au moins des traits pointillés en position 1(2), 6(7), 9(11) représente une double liaison pour la préparation de médicaments ayant une activité inhibitrice de l'aromatase.

**Claims**

1. The products of general formula (I):

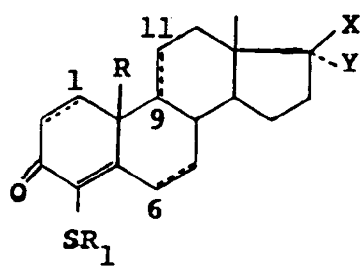

(I)

in which the R substituent represents an alkyl, alkenyl or alkynyl radical having at most 4 carbon atoms or a CN radical;
$R_1$ represents an alkyl radical having 1 to 6 carbon atoms substituted by one or more substituents from the group formed by alkyloxy or alkylthio radicals having 1 to 4 carbon atoms, nitro and cyano radicals and halogen atoms; the X and Y substituents are such that either they represent together an oxo group or X represents an optionally acylated or etherified hydroxy radical and Y represents a hydrogen atom, the dotted lines in position 1(2), 6(7) and 9(11) indicate the optional presence of a second bond between the carbons which carry them.

2. The products of general formula (I) as defined in claim 1 in which R represents a methyl radical and $R_1$ is chosen from the $CH_2Hal$ or $CHHal_2$ radicals in which Hal represents a halogen atom, $CH_2CN$, $CH_2OCH_3$, $CH_2SCH_3$ and $CH_2NO_2$.

3. The products of general formula (I) as defined in one of claims 1 or 2 in which $R_1$ is chosen from the $CH_2Cl$, $CH_2F$ or $CHF_2$ radicals and R represents a methyl radical.

4. The products of general formula (I) as defined in one of claims 1 to 3 and corresponding to the following formulae:
   - 4-[(fluoromethyl) thio] androst-4-ene-3,17-dione and
   - 4-[(chloromethyl) thio] androst-4-ene-3,17-dione.

5. Preparation process for the products of general formula (I) as defined in claim 1, characterized in that a halide of formula Hal'$R_1$ in which Hal' represents a halogen atom is reacted on a product of formula (II):

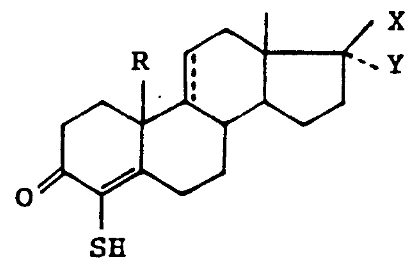

(II)

in order to obtain a product of formula ($I_A$):

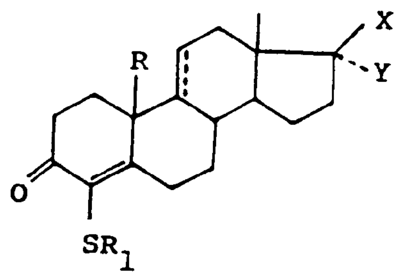

($I_A$)

corresponding to a product of formula (I) in which the dotted lines in position 1(2) and 6(7) represent a single bond between the carbons which carry them and <u>if desired</u> a dehydrogenation reagent is reacted on the product of formula ($I_A$) in order to obtain a product of formula ($I_B$):

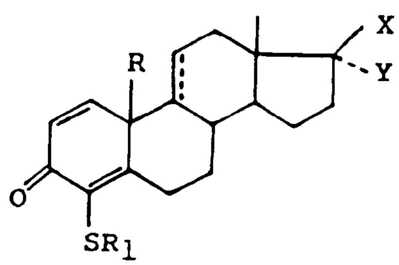

($I_B$)

corresponding to a product of formula (I) in which the dotted line in position 1(2) indicates the presence of a second bond between the carbons which carry it and the dotted line in position 6(7) represents a single bond and if desired firstly a protection reagent of the 3-keto delta-4 function then a dehydrogenation reagent is reacted on the products of formula ($I_A$) or ($I_B$) in order to obtain a product of formula ($I_C$):

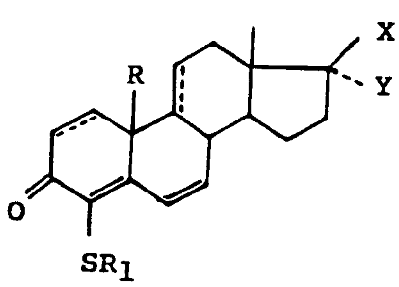

($I_C$)

corresponding to a product of formula (I) in which the dotted line in position 6(7) indicates the presence of a second bond between the carbons which carry it.

6. As medicaments, the products of general formula (I) as described in claim 1.

7. As medicaments, the products of general formula (I) as described in one of claims 2, 3 or 4.

8. The pharmaceutical compositions containing, as active ingredient, at least one of the medicaments defined in one of claims 6 or 7.

9. Use of the products of general formula (I):

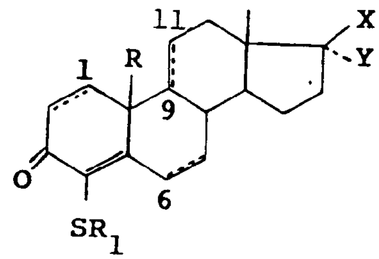

(I)

in which the R substituent represents an alkyl, alkenyl or alkynyl radical having at most 4 carbon atoms or a CN radical;
$R_1$ represents an alkyl radical having 1 to 6 carbon atoms non-substituted or substituted by one or more substituents from the group formed by alkyloxy or alkylthio radicals having 1 to 4 carbon atoms, nitro and cyano radicals and halogen atoms; the X and Y substituents are such that either they represent together an oxo group or X represents an optionally acylated or etherified hydroxy radical and Y represents a hydrogen atom, the dotted lines in position 1(2), 6(7) and 9(11) indicate the optional presence of a second bond between the carbons which carry them, it being understood that when $R_1$ represents a non-substituted alkyl radical and X and Y represent together an oxo group, at least one of the dotted lines in position 1(2), 6(7), 9(11) represents a double bond, for the preparation of medicaments having an aromatase inhibiting activity.

## Patentansprüche

1. Produkte der allgemeinen Formel (I)

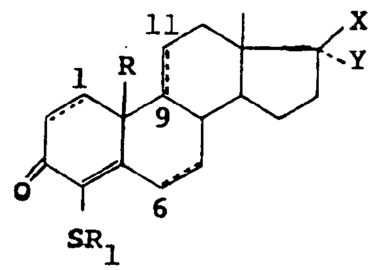

(I)

worin der Substituent R für einen Alkyl-, Alkenyl- oder Alkinylrest mit höchstens 4 Kohlenstoffatomen oder eine Gruppe CN steht;
$R_1$ einen Alkylrest mit 1 bis 6 Kohlenstoffatomen, substituiert durch einen oder mehrere Substituenten, ausgewählt unter den Alkoxy- oder Alkylthioresten mit 1 bis 4 Kohlenstoffatomen, Nitro, Cyano und den Halogenatomen bedeutet; die Substituenten X und Y derart sind, daß sie entweder zusammen eine Oxogruppe bilden oder X eine gegebenenfalls acylierte oder veretherte Hydroxygruppe bedeutet und Y für ein Wasserstoffatom steht, wobei die gestrichelten Linien in 1(2)-, 6(7)- und 9(11)-Stellung die etwaige Anwesenheit einer zweiten Bindung zwischen den sie aufweisenden Kohlenstoffen anzeigen.

2. Produkte der allgemeinen Formel (I) gemäß Anspruch 1, worin R für eine Methylgruppe steht und $R_1$ unter den Resten $CH_2Hal$ oder $CHHal_2$, worin Hal für ein Halogenatom steht, $CH_2CN$, $CH_2OCH_3$, $CH_2SCH_3$ und $CH_2NO_2$ ausgewählt ist.

3. Produkte der allgemeinen Formel (I) gemäß einem der Ansprüche 1 oder 2, worin $R_1$ unter den Resten $CH_2Cl$, $CH_2F$ oder $CHF_2$ ausgewählt ist und R für eine Methylgruppe steht.

4. Produkte der allgemeinen Formel (I) wie in einem der Ansprüche 1 bis 3 definiert, entsprechend den folgenden Bezeichnungen:
4-[(Fluormethyl)thio]-androst-4-en-3,17-dion und
4-[(Chlormethyl)thio]-androst-4-en-3,17-dion.

5. Verfahren zur Herstellung der Produkte der allgemeinen Formel (I) wie in Anspruch 1 definiert, dadurch gekennzeichnet, daß man ein Halogenid der Formel Hal'$R_1$, worin Hal' für ein Halogenatom steht, mit einem Produkt der Formel (II)

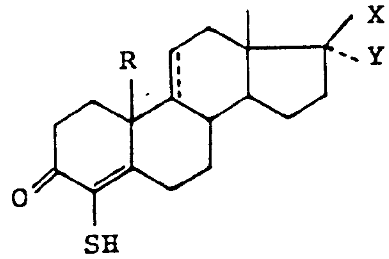

(II)

umsetzt, um zu einem Produkt der Formel ($I_A$)

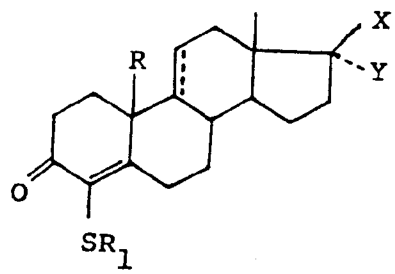

($I_A$)

entsprechend einem Produkt der Formel (I) zu gelangen, worin die gestrichelten Linien in 1(2)- und 6(7)-Stellung eine Einfachbindung zwischen den sie aufweisenden Kohlenstoffen wiedergeben, und gewünschtenfalls mit dem Produkt der Formel ($I_A$) ein Dehydrierungsreagens umsetzt, um zu einem Produkt der Formel ($I_B$)

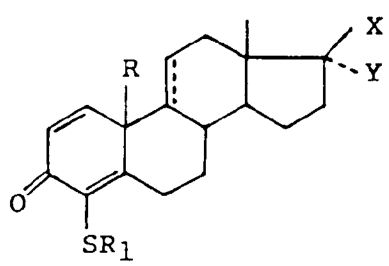

($I_B$)

entsprechend einem Produkt der Formel (I) zu gelangen, worin die gestrichelte Linie in 1(2)-Stellung die Anwesenheit einer zweiten Bindung zwischen den sie aufweisenden Kohlenstoffen anzeigt und die gestrichelt Linie in 6(7)-Stellung eine Einfachbindung wiedergibt, und gewünschtenfalls mit den Produkten der Formel ($I_A$) oder ($I_B$) zunächst ein Reagens für den Schutz der 3-Keto-Δ-4-Funktion, dann ein Dehydrierungsreagens umsetzt, um zu einem Produkt der Formel ($I_C$)

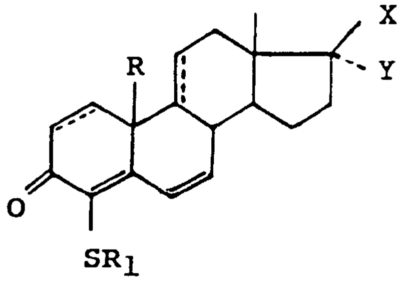

$(I_C)$

entsprechend einem Produkt der Formel (I) zu gelangen, worin die gestrichelte Linie in 6(7)-Stellung die Anwesenheit einer zweiten Bindung zwischen den sie aufweisenden Kohlenstoffen anzeigt.

**6.** Als Arzneimittel die Produkte der allgemeinen Formel (I), wie in Anspruch 1 beschrieben.

**7.** Als Arzneimittel die Produkte der allgemeinen Formel (I), wie in einem der Ansprüche 2, 3 oder 4 beschrieben.

**8.** Pharmazeutische Zusammensetzungen, enthaltend als Wirkstoff zumindest eines der Arzneimittel, wie in einem der Ansprüche 6 oder 7 definiert.

**9.** Verwendung der Produkte der allgemeinen Formel (I)

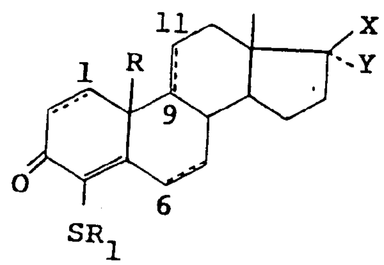

(I)

worin der Substituent R einen Alkyl-, Alkenyl- öder Alkinylrest mit höchstens 4 Kohlenstoffatomen oder eine Gruppe CN bedeutet;
$R_1$ für einen Alkylrest mit 1 bis 6 Kohlenstoffatomen, unsubstituiert oder substituiert durch einen oder mehrere Substituenten, ausgewählt unter den Alkoxy- oder Alkylthioresten mit 1 bis 4 Kohlenstoffatomen, Nitro , Cyano und den Halogenatomen, steht; die Substituenten X und Y derart sind, daß sie entweder gemeinsam eine Oxogruppe bilden oder X eine gegebenenfalls acylierte oder veretherte Hydroxygruppe bedeutet und Y ein Wasserstoffatom darstellt, die gestrichelten Linien in 1(2)-, 6(7)- und 9(11)-Stellung die etwaige Anwesenheit einer zweiten Bindung zwischen den sie aufweisenden Kohlenstoffen anzeigen, mit der Maßgabe, daß, wenn $R_1$ einen unsubstituierten Alkylrest bedeutet und X und Y zusammen eine Oxogruppe bilden, zumindest eine der gestrichelten Linie in 1(2)-, 6(7)-, 9(11)-Stellung eine Doppelbindung wiedergibt, für die Herstellung von Arzneimitteln mit einer die Aromatase inhibierenden Aktivität.